# EUROPÄISCHE PATENTSCHRIFT

(11) **EP 3 909 485 B1**
(45) Veröffentlichungstag und Bekanntmachung des Hinweises auf die Patenterteilung: **01.11.2023**
(21) Anmeldenummer: 20206763.3
(22) Anmeldetag: 10.11.2020
(51) Int. Cl.: A47K 7/00

(54) **VORRICHTUNG ZUR HANDREINIGUNG**
DEVICE FOR HAND CLEANING
DISPOSITIF POUR LE NETTOYAGE DES MAINS

(30) Priorität: 12.05.2020 DE 202020102666 U
(43) Veröffentlichungstag der Anmeldung: 17.11.2021
(73) Patentinhaber: Singer, Alexander, 87600 Kaufbeuren (DE)
(72) Erfinder: Singer, Alexander, 87600 Kaufbeuren (DE)
(74) Vertreter: Fleuchaus & Gallo Partnerschaft mbB

(56) Entgegenhaltungen:
- CN-Y- 2 599 009
- DE-A1- 10 315 720
- DE-U1-202007 008 528
- US-A- 6 039 938
- US-A1- 2007 260 202
- Zwilling: "Preisliste Küche und Bestecke", , Juni 2012 (2012-06), Seiten 1-208, XP055790224, Gefunden im Internet: URL:http://www.icono.com.mx/zwilling.pdf [gefunden am 2021-03-25] & Unknown: "Auszüge aus dem Internet", , 25. März 2021 (2021-03-25), Seiten 1-2, XP55790228, Gefunden im Internet: URL:https://www.amazon.com/Zwilling-J-Henc kels-Stainless/dp/B002DGTI0M [gefunden am 2021-03-25]

## Beschreibung

Die Erfindung betrifft eine Vorrichtung zur Reinigung mindestens einer Hand und insbesondere eine Vorrichtung zur Handreinigung mittels Reibung der Vorrichtung an mindestens einer Hand.

Mittel zur Handreinigung sind im Stand der Technik bekannt. Beispiele solcher Mittel sind handelsübliche Flüssig- und Festseifen, sowie Desinfektionstücher. Diese weisen jedoch unter Anderem den Nachteil auf, dass sie entweder selber Feucht sind oder nur im Zusammenspiel mit Feuchtigkeit einen wesentlichen Reinigungseffekt erzielen.

Sind die Mittel allgemein oder nach deren Verwendung feucht, wie beispielsweise bei Desinfektionstüchern und Festseifen, so können sie deren Umgebung in der sie transportiert werden (z.B. Taschen oder Hosentaschen) verschmutzen und schlimmstenfalls beschädigen. Die alltägliche Mitnahme ist somit beschränkt und geeignete Aufnahme- oder Transportbehälter werden benötigt. Sind die Mittle lediglich mit Wasser verwendbar, so können sie im Alltag nur eingeschränkt mitgeführt bzw. eingesetzt werden. Schließlich neigen Festseifen, wie zum Beispiel handelsübliche Seifenstücke, zum zerbröckeln, wenn sie nicht mit einer gewissen Vorsicht behandelt werden.

Die Wiederverwendungsmöglichkeiten der Vorrichtungen des Standes der Technik sind auch limitiert. Desinfektionstücher und Flüssigseife sind zum Beispiel jeweils nur einmal verwendbar, weswegen sie häufig ersetzt werden müssen. Das ist einerseits kostenintensiv und andererseits umweltschädigend, auch aufgrund des erheblichen Verpackungsaufwands für, z.B. Flüssigseifen.

Auch der Verbrauch von Festseifen ist bei deren Verwendung erheblich, ohne dass die verbrauchte Seife vollständig zur Handreinigung verwendet worden ist. Schließlich verringert sich die Größe der Festseife im Verlauf des Gebrauchs, so dass deren Anwendbarkeit immer schwieriger ist, bis hin zu einem Zeitpunkt an dem ein noch erheblicher Rest an Seifenmaterial nicht mehr zu einfach zu verwenden ist und somit häufig verworfen wird. Dies verursacht ebenfalls eine erhebliche Belastung der Umwelt.

Aus medizinischer Sicht ist bekannt, dass solche Vorrichtungen des Standes der Technik die Hautflora angreifen und hierdurch eine Vielzahl von unerwünschten Wirkungen auftreten können, wie z.B. Hauttrockenheit bis hin zur -rissigkeit, Ekzeme, und dergleichen.
US 6,039,938 A1 offenbart eine Vorrichtung zur Handreinigung aus Metall.
US 2007/0260202 A1 offenbart eine Vorrichtung zur Körperreinigung mit einer Außenfläche aus Metalloxid.

Vor diesem Hintergrund ist es die Aufgabe der vorliegenden Erfindung, eine Vorrichtung zur Handreinigung bereitzustellen, welche die Nachteile im Stand der Technik zumindest teilweise überwindet.

Diese Aufgabe wird durch eine Vorrichtung gemäß dem unabhängigen Anspruch 1 gelöst sowie durch eine Verwendung dieser Vorrichtung gemäß dem unabhängigen Anspruch 14. Bevorzugte Ausführungsformen sind Gegenstand der jeweiligen abhängigen Ansprüche.

Demnach wird eine Vorrichtung zur Handreinigung bereitgestellt, welche einen Körper mit einer Deckschicht aufweist. Die Deckschicht weist einen ersten Deckschichtbereich und einen zweiten Deckschichtbereich auf, wobei der erste und der zweite Deckschichtbereich zumindest teilweise einen allgemeinen konvexen Verlauf aufweisen. Ferner definiert der erste Deckschichtbereich eine Vertiefung.

Der erste Deckschichtbereich und der zweite Deckschichtbereich sind außerdem aus einem ersten antimikrobiellen Edelmetall bzw. zweiten antimikrobiellen Edelmetall gefertigt. Der Körper ist aus einem dritten Material gefertigt, welches keine erheblichen Mengen antimikrobieller Edelmetalle enthält.

Eine Vorrichtung zur Handreinigung ist insbesondere eine Vorrichtung, die mit mindestens einer Hand in Kontakt gebracht werden kann, um die Handflächen sowie ggf. auch die Finger, Fingerkuppen und Zwischenfingerbereiche zu reinigen. Dabei kann die Vorrichtung einzeln an jeder Hand verwendet werden, oder zusammen gleichzeitig für beide Hände. Bevorzugt erfolgt die Reinigung durch ein Reiben der Vorrichtung innerhalb der Hand oder zwischen den Händen sowie an den Fingern, etc.

Selbstverständlich ist die erfindungsgemäße Vorrichtung nicht auf die Reinigung von Händen beschränkt und kann gegebenenfalls auch zur Reinigung anderer Körperteile verwendet werden.

Der Körper der Vorrichtung weist eine Deckschicht auf; bei einer Ausführungsform der Erfindung umgibt die Deckschicht den Körper dabei im Wesentlichen vollständig, so dass ein Kontakt der Vorrichtung mit der Haut stets nur mit dem(n) Deckschichtmaterial(ien) erfolgt.

Gemäß einer weiteren Ausführungsform der erfindungsgemäßen Vorrichtung ist der Körper mit der Deckschicht über eine Trägerschicht verbunden. Hierdurch kann für bestimmte Materialkombinationen des Körpers bzw. der Deckschicht eine verbesserte Bindung erzielt werden, so dass sichergestellt ist, dass auch bei langandauernder Verwendung keine Abhebung der Deckschicht vom Körper erfolgt. Besonders bevorzugt kommen solche Trägerschichten bei der Verwendung von nicht-metallischen Materialien für den Körper zum Einsatz.

Ferner weist die Deckschicht einen ersten und einen zweiten Deckschichtbereich auf, wobei diese zwei Bereiche die gesamte Deckschicht oder auch nur ein Teilbereich der gesamten Deckschicht bilden können.

Der zumindest teilweise allgemein konvexe Verlauf des ersten und zweiten Deckschichtbereichs ist besonders vorteilhaft, da hierdurch zumindest bereichsweise die Form der Handfläche einer greifenden Hand entsprochen wird, so dass die Aufnahme mit der Hand vereinfacht und die Kontaktfläche zur Hand maximiert wird. Darüber hinaus ist das Gefühl für die aufnehmende Hand natürlich und angenehm, was dazu führt, dass der Körper länger in der Hand verbleibt. Dies erhöht wiederum die Reinigungswirkung unabhängig davon in welcher Orientierung die Vorrichtung in die Hand bzw. die Hände genommen wird.

Die durch den ersten Deckschichtbereich definierte Vertiefung ist dazu geeignet, dass eine oder mehrere Fingerkuppen hierin eingreifen können und hierdurch ein im Wesentlichen allseitiger Kontakt der Fingerkuppe mit dem ersten Material des ersten Deckschichtbereichs erzielbar ist. Die Tiefe der Vertiefung kann dabei so gewählt sein, dass beispielsweise die Aufnahme des ersten Fingerglieds eines Fingers weitgehend ermöglicht wird oder aber lediglich der äußerste Teil der Fingerkuppe das erste Material des ersten Deckschichtbereichs berührt.

Das erste und zweite Material aus dem der erste bzw. der zweite Deckschichtbereich gefertigt sind, weist jeweils ist jeweils ein erstes bzw. zweites antimikrobielles Edelmetall. Antimikrobielle Edelmetalle weisen üblicherweise einen oligodynamischen Effekt auf, wodurch sie antibakterielle, antivirale und antimykotische Wirken zeigen.

Bei der Verwendung der erfindungsgemäßen Vorrichtung durch Reiben in der Hand erfolgt somit im Wesentlichen durch den Kontakt z.B. der Handoberfläche mit dem ersten und/oder dem zweiten Deckschichtbereich und insbesondere durch dem Kontakt mit den ersten und zweiten antimikrobiellen Edelmetallen eine antimikrobielle und/oder antibakterielle Wirkung. Mikroskopisch kommt es bei der Verwendung zu einem geringfügigen Abrieb des ersten und zweiten Materials, durch welchen mikroskopische Partikel des im ersten und zweiten Material vorhandenen Edelmetalls an der Haut anhaften und dort ihren oligodynamischen Effekt und damit ihre antibakterielle, antivirale und antimykotische Wirkung entfalten.

Auch wenn nicht alle Hautflächen direkt mit dem ersten oder zweiten Deckschichtbereichs in Kontakt kommen, können sich die Edelmetallpartikel durch das nachträgliche Reiben der Hände miteinander weiter verteilen und deren Antimikrobielle Wirkung somit größerflächig entfalten.

Da bei derVerwendung lediglich mikroskopische Partikel des ersten und zweiten Materials abgerieben werden ist der Deckschichtabtrag minimal und die erfindungsgemäße Vorrichtung daher äußerst lange und widerholt verwendbar, ohne dass es zu einem merklichen Materialabtrag kommt. Die Größe der Vorrichtung bleibt somit trotz vielfacher Anwendungen im Wesentlichen unverändert; die Verwendbarkeit bleibt uneingeschränkt erhalten, auch über einen Zeitraum von Jahren.

Durch das Reiben der Vorrichtung in den Händen oder aber auch durch die Aufnahme der Vorrichtung in beispielsweise Taschen, wie z.B. Hosentaschen oder dergleichen, kommt es ferner zu einem kontinuierlichen Abrieb geringer Mengen des ersten und zweiten Materials vom ersten und/oder zweiten Deckschichtbereich, so dass hierdurch der Bildung von Oxidationsschichten auf dem ersten und/oder zweiten Deckschichtbereich zumindest teilweise entgegen gewirkt wird. Der Bildung solcher Oxidationsschichten kann auch durch das Reiben der Vorrichtung an einem Tuch oder einem Gewebe entgegengewirkt werden.

Die antimikrobielle Wirksamkeit des ersten und/oder zweiten Materials ist verbessert, wenn das enthaltene Edelmetall nicht oxidiert ist. Die antimikrobielle Wirksamkeit von nicht oxidiertem Kupfer gegen Escherichia Coli ist beispielsweise höher als die von oxidiertem Kupfer.

Vorteilhafterweise kann die erfindungsgemäße Vorrichtung trocken verwendet werden, wodurch sie leicht und bedenkenlos - also ohne sie mit besonderer Vorsicht zu behandeln - transportiert werden kann, beispielsweise in Taschen oder sogar Hosentaschen.

Die Vorrichtung kann aber auch mit Wasser verwendet werden, wobei dabei vorteilhaft ist, dass es zu einer verbesserten Verteilung der mikroskopischen Partikel des im ersten und zweiten Material vorhandenen Edelmetalls kommt. Schließlich lässt sich die Vorrichtung nach der Anwendung leicht trocknen bevor sie beispielsweise wieder in eine Handtasche oder eine Hosentasche verstaut wird.

Der Körper der erfindungsgemäßen Vorrichtung selbst ist aus einem dritten Material gefertigt, welches keine erhebliche Menge an antimikrobiellen Edelmetallen aufweist. Das dritte Material aus dem der Körper gefertigt ist, kann beispielsweise weitgehend Zink enthalten und im ZinkDruckguss hergestellt werden. Alternative hierzu könnte das dritte Material auch kunststoffbasiert sein und der Köper im Spritzguss hergestellt werden. Dies ist vorteilhaft, da Edelmetalle teuer und mitunter schwieriger zu verarbeiten sind. Der Körper kann entsprechend aus einem leicht zu verarbeitenden und verhältnismäßig günstigen Material gefertigt sein, wobei die gewünschte Reinigungswirkung durch den Deckschichtüberzug des Körpers erzielt wird.

Gemäß einer Ausführungsform der Erfindung ist die Deckschicht und insbesondere der erste und/oder der zweite Deckschichtbereich aus einem galvanisch abgeschiedenen ersten bzw. zweiten antimikrobiellen Edelmetall gefertigt. Die galvanische Abscheidung kann mittels eines cyanidischen Bads, z. B. eines cyanidischen Kupferbads, welches Oxidationshemmer enthält, erfolgen.

Die durch Oxidation eines galvanisch abgeschiedenen Metalls entstehenden Oxidschichten weisen eine vergleichsweise reduzierte Haftung auf, so dass ggf. an der Vorrichtung entstehende Oxidschichten am ersten und/oder zweiten Deckschichtbereich relativ leicht durch Reibung abgetragen werden können, beispielsweise, wie oben beschrieben, lediglich durch das Tragen der Vorrichtung in einer Hosentasche.

Diese reduzierte Haftung ist auf das Gefüge des galvanisch abgeschiedenen Metalls zurückführbar. Beispielsweise weist eine galvanisch abgeschiedene Kupferschicht eine ausgeprägte Zwillingsstruktur mit Zwillingsorientierungen auf. Insbesondere weist das Gefüge einer galvanisch abgeschiedenen Kupferschicht multiple Zwillingsbildung und Verwachsungen auf, welche eine solche galvanisch abgeschiedene Kupferschicht charakterisieren.

Die Oxidationsbeständigkeit des galvanisch abgeschiedenen Metalls ist somit erhöht, wodurch die antibakterielle Wirkung des ersten und zweiten Deckschichtbereichs und somit der erfindungsgemäßen Vorrichtung erhöht wird.

Gemäß einer Ausführungsform der erfindungsgemäßen Vorrichtung weist die Deckschicht eine Dicke auf, welche in einem Bereich zwischen 2µm bis 15µm, insbesondere in einem Bereich zwischen 3µm bis 13µm, insbesondere in einem Bereich zwischen 4µm bis 11µm liegt, insbesondere in einem Bereich zwischen 5µm bis 9µm liegt.

Diese Schichtdicken sind so gewählt, dass bei vergleichsweise Kostensparender Herstellung der erfindungsgemäßen Vorrichtung dennoch eine ausreichende Schichtdicke gewährleistet ist, so dass die Vorrichtung über einen langen Zeitraum ohne erhebliche Beschränkung der Wirkung nutzbar ist.

Die Vertiefung des ersten Deckschichtbereichs ist im Rahmen der Erfindung durch eine zumindest teilweise Abweichung des Verlaufs der Deckschicht im ersten Deckschichtbereich von der allgemein konvexen Form definiert. In Bezug auf den allgemein konvexen verlauf weist die Vertiefung des ersten Deckschichtbereichs somit einen allgemein konkaven Verlauf auf.

Beispielsweise kann die Vertiefung eine ebene Fläche des ersten Deckschichtbereichs sein, was insbesondere bei der Lagerung dervorrichtung vorteilhaft ist (eine rein konvexe Form führt zu einer instabileren Lagerung). Eine abgeflachte Vertiefung der ersten Deckschicht ist auch für eine dekorative Gravur sehr geeignet, so dass die Vorrichtung auch optisch ansprechend oder auch personalisiert werden kann.

Bei einer noch stärkeren Abweichung ist die Vertiefung als konkave Aussparung ausgeführt, was zu einer trogförmigen Vertiefung führt, in die beispielsweise Fingerkuppen eingreifen und hierin durch Reibung gereinigt werden können. Wird darüber hinaus beispielsweise mit den Fingerkuppen eine geeignete Kraft auf die Seitenwände der bevorzugt trogförmigen Vertiefung ausgeübt, kann die erfindungsgemäße Vorrichtung hierdurch innerhalb der Handfläche bewegt werden. Das daraus resultierende Reiben zwischen der Handfläche und der Vorrichtung, sowie zwischen den Fingerkuppen und der Vorrichtung führen dann zur gewünschten Reinigungswirkung der Vorrichtung.

Erfindungsgemäß weist die Vertiefung und insbesondere die allgemein konkave Vertiefung lediglich kontinuierliche Krümmungsradien auf. Dies ist von Vorteil, da hierdurch keine Toträume vorhanden sind, in denen sich eine ausgeprägte Oxidschicht ausbilden kann. Würde die Vertiefung Ecken oder spitze Winkel aufweisen, so wäre ein Erreichen dieser Ecken mittels eines Fingers bzw. einer Fingerspitze oder auch mittels eines textilen Abriebtuchs oder dergleichen zumindest erschwert, wodurch der Abrieb der Oxidschicht in diesen Bereichen vermindert wäre. Die antimikrobielle Wirkung der Vorrichtung könnte hierdurch verschlechtert sein. Es ist damit von besonderem Vorteil, wenn die Vertiefung eine allgemein konkave form aufweist und lediglich kontinuierliche Krümmungsrad, so dass beispielsweise mittels einer Fingerspitze sämtliche Bereiche der Vertiefung erreicht werden können und hierdurch ein Abrieb innerhalb der gesamten Vertiefung erzielt wird, was einerseits zu einer zuverlässigeren Reinigung führt und andererseits zur Verringerung der Ausbildung einer Oxidschicht in Bereich der Vertiefung. Bevorzugt ist das erste antimikrobielle Edelmetall und/oder das zweite antimikrobielle Edelmetall aus einer Gruppe ausgewählt, welche Silber, Gold, Kupfer und Legierungen dieser Metalle enthält. All diese Edelmetalle sind unter anderem bei Kontakt mit der Haut nicht Hautschädigend, wodurch auch die häufige Verwendung der Vorrichtung sehr Hautverträglich ist - im Gegensatz zu vielen Reinigungsvorrichtungen des Standes der Technik.

Kupfer wirkt, zum Beispiel, antibakteriell gegen eine Vielzahl von Bakterien und weist sogar antivirale Wirkungen, zum Beispiel gegen Humane-Noroviren, auf. Diese antimikrobielle und antivirale Effekte werden insbesondere durch die Abscheidung von Kupfer-Ionen und deren Kontakt mit Bakterien und Viren hervorgerufen. Ferner weist Kuper eine marginale, wenn nicht sogar nicht vorhandene Auswirkung auf die nicht-pathogen Bakterien der Hautflora auf, wodurch die Hautflora sogar geschützt wird, da Pathogene im relativen zahlenvergleich vermindert werden.

In ähnlicher Weise zeigt auch Silber antimikrobielle und antibakterielle Wirkungen, da sich Silber auf die Permeabilität von Zellmembranen auswirkt. Darüber hinaus hat Silber auch antivirale Effekte.

Gold weist zudem auch immunsuppressive und entzündungshemmende Wirkungen auf. Zudem regt Gold die Kollagenproduktion der Haut an, was der Hautgesundheit zuträglich ist.

Bei einer bevorzugten Ausführungsform der Erfindung sind das erste und das zweite Material gleich.

Durch die Wahl des dritten Materials, also des Materials des Körpers, kann die gemittelte Dichte der erfindungsgemäßen Vorrichtung stark beeinflusst werden, so dass eine Ausführungsform der erfindungsgemäßen Vorrichtung trotz der Verwendung einer Metallischen Deckschicht dennoch ein relativ geringes Gesamtgewicht aufweisen kann, welches insbesondere in einem Bereich zwischen 55g und 85g, zwischen 60g und 80g und bevorzugt zwischen 65g bis 75g liegt.

Hierdurch kann einerseits die strukturelle Stabilität des Körpers gewährleistet werden, die eine einfache Handhabung der Vorrichtung mit wenigstens einer Hand ermöglicht. Andererseits ermöglicht das relativ geringe Gewicht der Vorrichtung ein dauerhaftes Mitführen derselben beispielswiese in einer Handtasche oder einer Hosentasche, so dass eine häufige Anwendung durch den Benutzer im Tagesverlauf möglich ist.

Bevorzugt weist das dritte Material des Körpers eine Zinklegierung auf. Die Zinklegierung kann Zink, Aluminium und Kupfer enthalten, beispielsweise zwischen 3.5 Gew-% und 4.5 Gew.-% Aluminium, zwischen 0.5 Gew-% und 1.5 Gew.-% Kupfer, und als wesentlichen Rest Zink. Eine solche Zinklegierung ist besonders für den Einsatz beim Zinkdruckguss-Verfahren geeignet, da das Risiko der Entstehung von sogenannter Zinkpest verringert wird.

Allerdings ist die Menge an Kupfer in dieser Legierung zu gering, dass ein relevanter antimikrobieller oder antibakterieller Effekt darauf zurück zu führen währe; es handelt sich insoweit um eine im Sinne der Erfindung nicht erhebliche Menge an antimikrobiellem Edelmetall.

Die erfindungsgemäße Vorrichtung kann eine Vielzahl von für den Komfort auf der Hand geeignete Formen aufweisen. Gemäß einer Ausführungsform der erfindungsgemäßen Vorrichtung weist der Körper in einer ersten Ebene einen Querschnitt auf, welcher eine erste Richtung und eine hierzu orthogonale zweite Richtung hat und bei dem die Länge der ersten Richtung dabei länger als die Länge der zweiten Richtung.

Besonders bevorzugt weist der Körper in der ersten Ebene einen Querschnitt auf, der eine ovale Form und bevorzugt eine spitz-ovale Form aufweist.

Besonders bevorzugt ist das Verhältnis *q* der Länge der zweiten Richtung zur Länge der ersten Richtung, gemäß einer weitere Ausführungsform, 0,2 < *q* < 0,5, vorzugsweise 0,3 < *q* < 0,4, und bevorzugt *q* ≈ 0,35.

In Ausführungsform weist der Körper eine Größe in der ersten Richtung auf, welche in einem Bereich zwischen 70mm und 90mm, und insbesondere in einem Bereich zwischen 75mm und 85mm liegt. In der zweiten Richtung weist der Körper eine Größe auf, welche in einem Bereich zwischen 20mm und 38mm, und insbesondere in einem Bereich zwischen 23mm bis 35mm liegt.

Die relativ kleinen Abmessungen der erfindungsgemäßen Vorrichtung machen sie relativ leicht zu transportieren, was die Benutzung der Vorrichtung jederzeit und/oder nach einem Kontakt mit kontaminierten Oberflächen ermöglicht. Beispielsweise kann die Vorrichtung, ebenso wie auch durch das geringe Gewicht begünstigt, relativ leicht in einer Hosentasche oder Handtasche mitgeführt werden. In diesen Fall wird die Bildung von Oxidationsschichten durch das Reiben der Vorrichtung am Gewebe, bspw. der Hosentasche, verringert bzw. schon gebildete Oxidationsschichten kontinuierlich abgerieben.

Bei einer weiteren Bevorzugten Ausführungsform weist der Körper in wenigstens einer weiteren Ebene, bevorzugt bei 2 weiteren, im Wesentlichen orthogonal zueinander orientierten Ebenen, welche im Wesentlichen orthogonal zur ersten Ebene sind, einen trogförmigen Querschnitt auf. Die so definierte Trogform bildet dabei die durch den ersten Deckschichtbereich definierte Vertiefung aus.

Die so definierte Form ist besonders vorteilhaft beim Ablegen und festhalten der Vorrichtung.

Der erste Deckschichtbereich und der zweiten Deckschichtbereich können dabei im Allgemeinen einander gegenüberliegend angeordnet sein, so dass beim Greifen der Vorrichtung mit einer Hand sowohl mindestens ein Abschnitt des ersten Deckschichtbereichs und mindestens ein Abschnitt des zweiten Deckschichtbereichs mit jeweiligen Bereichen der Handfläche in Kontakt kommen.

In einer weiteren Ausführungsform erstreckt sich der Körper entlang der ersten Richtung longitudinal und die Vertiefung ist in einer hierzu im Wesentlichen parallelen Richtung langgestreckt. Durch diese langestreckte Gestaltung der Vertiefung ist es möglich, das gleichzeitig mehrere Finger in die Vertiefung eingreifen, was sowohl einen verbesserten Griff der Vorrichtung ermöglicht als auch ein gleichzeitiges Reinigen mehrerer Fingerkuppen.

Bevorzugt weist der Körper der erfindungsgemäßen Vorrichtung eine Form auf, welche langgestreckt linsenförmig oder langestreckt ellipsoidal ist, und vorzugsweise allgemein spindelförmig ist. Diese Formen ermöglichen ein ergonomisches Greifen des Körpers mit der Hand und vergrößern die Kontaktfläche zwischen der Hand und dem Körper, was die Handdesinfektion Wirkung der erfindungsgemäßen Vorrichtung erhöht.

Insbesondere aber hat ein so geformter Körper einer Ausführungsform der Erfindung die charakteristische Form eines Handschmeichlers, so dass wiederum hierdurch der Benutzer die Vorrichtung häufiger greift und reibend in der Hand bewegt.

Eine häufige Anwendung der Vorrichtung der Erfindung erhöht deren antibakterielle und antimikrobielle Wirkung bei der Handreinigung.

Nachfolgend werden verschieden Ausführungsformen der Erfindung erläutert, wobei diese die Erfindung nur Beispielshaft erläutern und keine Einschränkung des allgemeinen Erfindungsgedankens in Bezug auf Abwandlungen darstellen. Dabei zeigt:
- **Fig. 1**: eine schematische dreidimensionale Darstellung einer Ausführungsform der erfindungsgemäßen Vorrichtung;
- **Fig. 2**: eine schematische Draufsicht der Vorrichtung aus Figur 1;
- **Fig. 3**: eine schematische Frontansicht der Vorrichtung aus Figur 1;
- **Fig. 4**: eine schematische untere Ansicht der Vorrichtung aus Figur 1;
- **Fig. 5**: ein Querschnitt der Ausführungsform der erfindungsgemäßen Vorrichtung; und
- **Fig. 6**: ein Längsschnitt derAusführungsform der erfindungsgemäßen Vorrichtung.

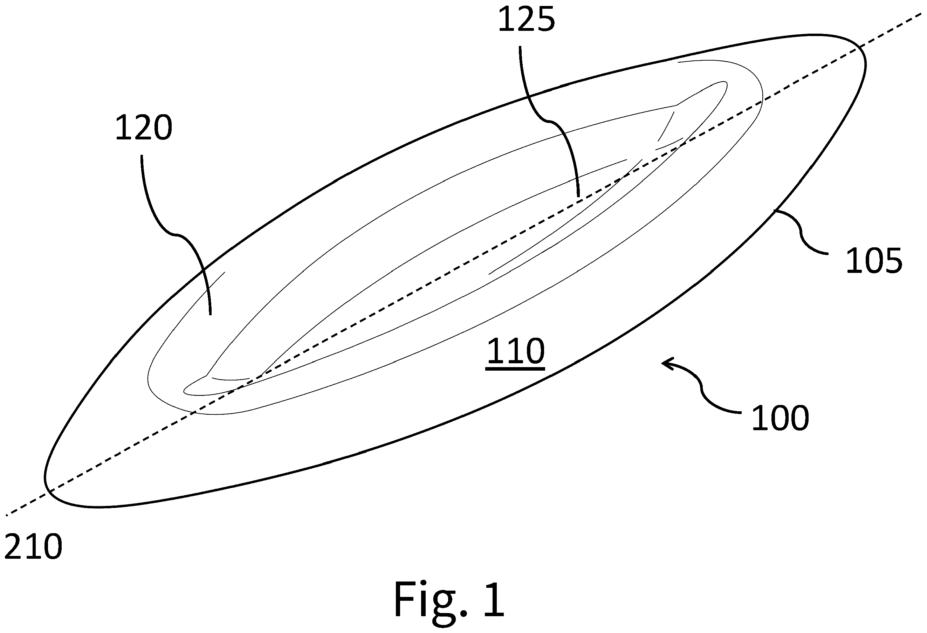

Die in der **Figur 1** schematisch dargestellte Ausführungsform der erfindungsgemäßen Vorrichtung weist einen Körper 105 mit einer Deckschicht 110 auf, welche vorliegend den gesamten Körper 105 der Vorrichtung 100 umgibt. Dementsprechend ist der Körper in dieser Ansicht nicht direkt sichtbar sondern lediglich die den Körper umgebende Deckschicht 110.

Die Deckschicht 110 weist ferner einen ersten Deckschichtbereich 120 und einen zweiten Deckschichtbereich auf, wobei lediglich der erste Deckschichtbereich in dieser Figur dargestellt ist. Der erste und der zweite Deckschichtbereich verlaufen im Wesentlichen konvex und sind in dieser Ausführungsform gegenüberliegend angeordnet.

Die Vorrichtung 100 bzw. der Körper 105 erstreckt sich länglich entlang der ersten Achse 210 und weist eine spitz-Ovale Form auf.

Der erste Deckschichtbereich 120 definiert eine Vertiefung 125 die sich parallel zu der ersten Achse 210 erstreckt. Die Vertiefung 125 weist im Wesentlichen kontinuierliche Krümmungsradien auf, so dass beispielsweise mittels einer Fingerspitze sämtliche Bereiche der Vertiefung 125 erreicht werden können.

Der erste Deckschichtbereich 120 und derzweite Deckschichtbereich sind aus einem ersten bzw. einem zweiten antimikrobielles Edelmetall gefertigt, wie beispielsweise Kupfer. Der Körper hingegen ist aus einem dritten Material gefertigt, welches keine erheblichen Mengen antimikrobieller Edelmetalle enthält, wie beispielsweise Zink.

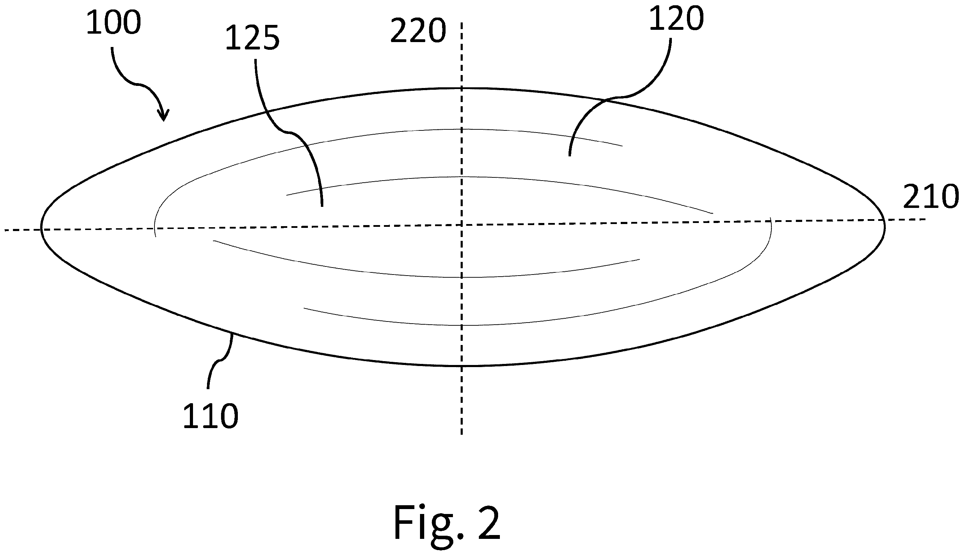

**Figur 2** zeigt eine schematische Draufsicht der Vorrichtung 100 gemäß Figur 1, so dass der erste Deckschichtbereich 120 zu sehen ist. Die Vorrichtung 100 erstreckt sich in Richtung der ersten Achse 210 länger als in Richtung der zweiten Achse 220, welche zu der ersten Richtung senkrecht verläuft. Das Verhältnis *q* der Länge der Vorrichtung in Richtung der zweiten Achse 220 zur Länge in Richtung der ersten Achse 210 liegt vorliegend bei *q* ≈ 0,35.

Die Erstreckung der Vertiefung 125 erfolgt im Wesentlichen symmetrisch zu der Erstreckung der Vorrichtung und entsprechend erstreckt sich die Vertiefung 125 länger entlang der ersten Achse 210 als der zweiten Achse 220.

Ein Greifen der Vorrichtung 100 mit der Hand, so dass die Fingerspitzen der Hand im Wesentlichen senkrecht zu der ersten Achse 210 in die Vertiefung eingreifen, ermöglicht eine gute Handhabung der Vorrichtung 100,da hierdurch ein im Wesentlichen allseitiger Kontakt der Fingerkuppen mit dem ersten Material des ersten Deckschichtbereichs 120 erreicht wird.

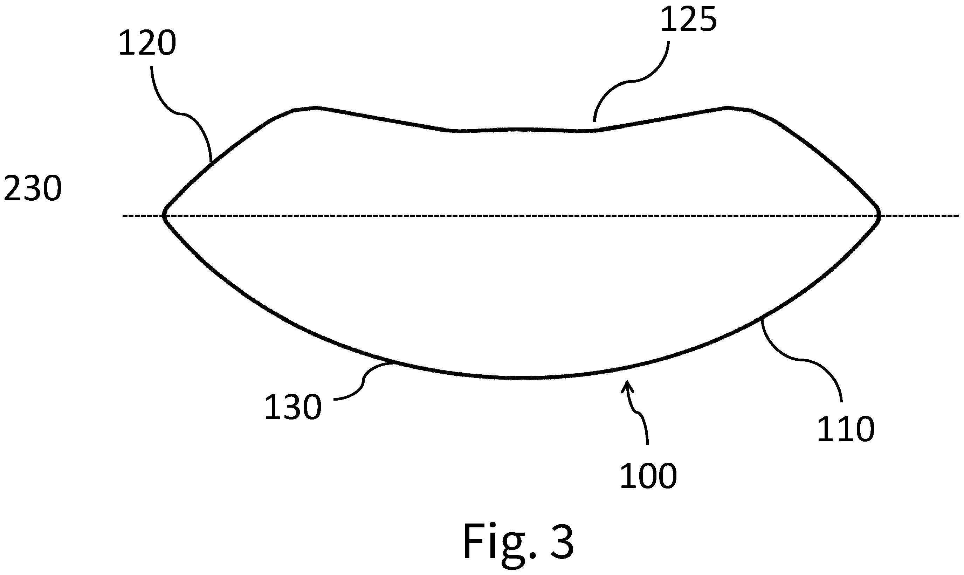

**Figur 3** zeigt eine Frontalansicht der Vorrichtung 100 entlang der ersten Achse 210 aus den vorherigen Figuren.

Eine dritte Achse 230 unterteilt die Deckschicht 110 in den ersten und zweiten Deckschichtbereich 120 und 130, welche einander gegenüberliegend angeordnet sind. Der erste Deckschichtbereich 120 definiert die Vertiefung 125, welche durch eine Abweichung des allgemeinen konvexen Verlaufs der ersten Deckschicht 120 definiert ist. Insbesondere ist in dieser Ausführungsform die Vertiefung 125 als eine konkave Aussparung ausgeführt.

Die Vertiefung 125 weist im Wesentlichen kontinuierliche Krümmungsradien auf, so dass beispielsweise mittels einer Fingerspitze sämtliche Bereiche der Vertiefung 125 erreicht werden können und eine Reinigung der Fingerspitzen erfolgt. Ferner wird somit eine auf der Deckschicht innerhalb der Vertiefung sich bildende Oxidschicht leicht durch die Fingerspitzen abgerieben.

Die Vorrichtung 100 weist an ihrer unteren Seite also an der Seite, welche den zweiten Deckschichtbereichs 130 aufweist, eine im Wesentlichen konvexe Bogenform auf, so dass ein Greifen der Vorrichtung 100 mit der Hand geschmeidig ist. Allgemein weist die Vorrichtung eine handschmeichelnde Form auf, wodurch deren häufige Benutzung gefördert wird.

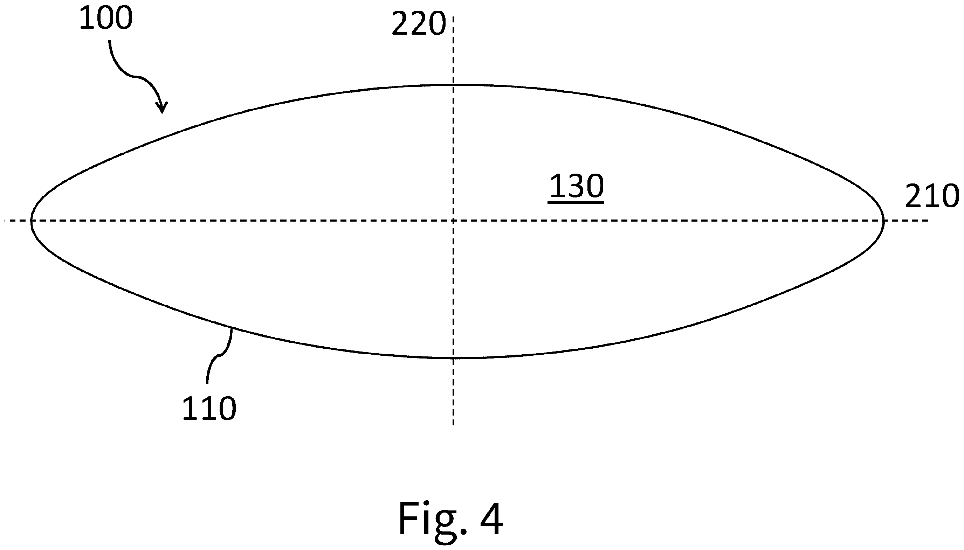

**Figur 4** zeigt eine schematische untere Ansicht der Vorrichtung 100 aus Figur 1. Dabei ist lediglich der zweite Deckschichtbereich 130 zu sehen. Die Vorrichtung 100 weist eine spitz-ovale Form auf, welche in Richtung der ersten Achse 210 langgestreckt ist. Die Oberfläche des zweiten Deckschichtbereichs 130 weist keine Aussparung oder Vertiefung auf, und ist im Wesentlichen konvexen, was dieser Figur jedoch nicht entnehmbar ist.

Bei der Aufnahme der Vorrichtung 100 mit einer Hand maximiert sich die Kontaktfläche zwischen dem zweiten Deckschichtbereich 130 und der Handoberfläche, so dass ein bestmöglichen Reinigungseffekt erzielt wird.

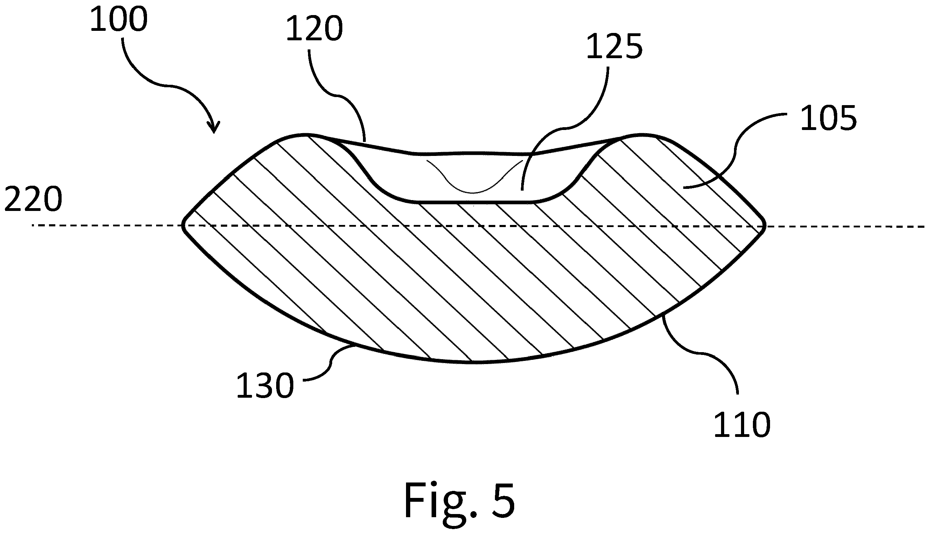

In **Figur 5** wird ein Querschnitt einer Ausführungsform der erfindungsgemäßen Vorrichtung 100 gezeigt. Die Vorrichtung 100 weist dabei einen Körper 105 auf, welcher aus einem dritten Material gefertigt ist, und welches keine erhebliche Mengen an antimikrobiellen Edelmetallen aufweist.

Der Körper 105 ist von einer Deckschicht 110 komplett umhüllt, wobei sich die Deckschicht 110 in einen ersten und einen zweiten Deckschichtbereich 120, 130 aufteilt ist, und wobei die Deckschichtbereiche in Bezug auf die zweite Achse 220 gegenüberliegen angeordnet sind. Der von dem zweiten Deckschichtbereich 130 umhüllte Abschnitt des Körper 105 weist im Querschnitt eine im Wesentlichen halb-ovale Form auf, so dass der zweite Deckschichtbereich 130 einen konvexen Verlauf aufweist.

Der erste Deckschichtbereich 120 hingegen definiert eine Vertiefung 125, so dass der von dem ersten Deckschichtbereich 120 umhüllte Abschnitt des Körper 105 einen im Wesentlichen trogförmigen Querschnitt definiert.

Der erste und zweite Deckschichtbereich 120, 130 sind in dieser beispielhaften Ausführungsform aus dem gleichen Material gefertigt, insbesondere aus einem galvanisch abgeschiedenen Edelmetall, zum Beispiel Kupfer, gefertigt. Die Deckschicht weist bevorzugt eine Dicke von ca. 8µm auf.

Das Reiben der Vorrichtung an die Hautoberfläche und insbesondere den Flächen der Hand und er Finger, bewirkt einen Kontakt des ersten und/oder zweiten Deckschichtbereichs 120, 130 mit der Hautoberfläche, wodurch mikroskopische Partikel des im ersten und zweiten Material vorhandenen Edelmetalls an der Haut anhaften und dort ihren oligodynamischen Effekt und damit ihre antibakterielle, antivirale und antimykotische Wirkung entfalten .

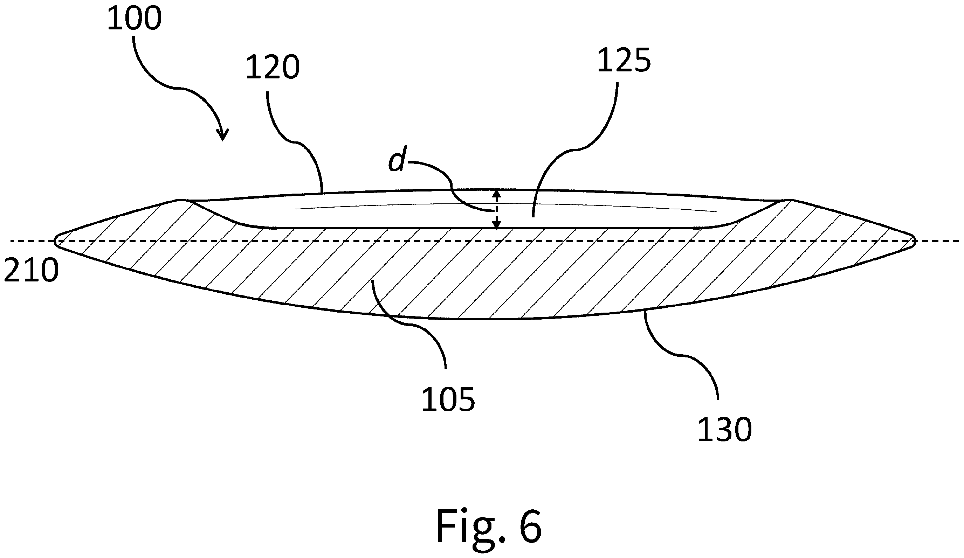

**Figur 6** zeigt einen seitlichen Querschnitt der Ausführungsform der Vorrichtung 100 aus Figur 5. Insbesondere definiert die erste Deckschicht 120 eine Vertiefung 125, welche sich Entlang der ersten Achse 210 länglich erstreckt. Die Tiefe d der Vertiefung 125 ist in dieser beispielhaften Ausführungsform so gewählt das zumindest eine Fingerkuppe einer Hand in die Vertiefung eingreifen kann und dort durch ein Verschieben innerhalb der Vertiefung 125 gereinigt werden kann.

### Bezugszeichenliste

100 Vorrichtung
105 Körper
110 Deckschicht
120 erster Deckschichtbereich
125 Vertiefung
130 zweiter Deckschichtbereich
210 erste Achse
220 zweite Achse
230 dritte Achse
*d* Tiefe der Vertiefung

## Patentansprüche

1. Vorrichtung (100) zur Handreinigung aufweisend einen Körper (105) mit einer Deckschicht (110), wobei die Deckschicht (110) einen ersten Deckschichtbereich (120) und einen zweiten Deckschichtbereich (130) aufweist, und der erste und der zweite Deckschichtbereich (120,130) zumindest teilweise einen allgemeinen konvexen Verlauf aufweisen, und wobei der erste Deckschichtbereich eine Vertiefung (125) definiert,
**dadurch gekennzeichnet, dass**
der erste Deckschichtbereich (120) und der zweite Deckschichtbereich (130) aus einem ersten antimikrobiellen Edelmetall beziehungsweise zweiten antimikrobiellen Edelmetall gefertigt sind, und
der Körper (105) aus einem Material gefertigt ist, welches keine erheblichen Mengen antimikrobieller Edelmetalle enthält,
wobei die durch den ersten Deckschichtbereich (120) definierte Vertiefung (125) durch eine Abweichung vom allgemein konvexen Verlauf des ersten Deckschichtbereichs (120) definiert ist, und vorzugsweise der erste Deckschichtbereich (120) im Bereich der Vertiefung (125) einen allgemein konkaven Verlauf aufweist, und lediglich kontinuierliche Krümmungsradien aufweist.

2. Vorrichtung nach Anspruch 1, wobei die Deckschicht (110) eine Dicke aufweist, welche in einem Bereich zwischen 2µm und 15µm liegt, vorzugsweise in einem Bereich zwischen 3µm und 13µm liegt, weiter vorzugsweise in einem Bereich zwischen 4µm und 11µm liegt, und bevorzugt in einem Bereich zwischen 5µm bis 9µm liegt.

3. Vorrichtung (100) nach einem der vorstehenden Ansprüche, wobei der Körper (105) mit der Deckschicht (110) mittels einer Trägerschicht verbunden ist.

4. Vorrichtung (100) nach einem der vorstehenden Ansprüche, wobei die Deckschicht (110) den Körper (105) im Wesentlichen vollständig umgibt.

5. Vorrichtung (100) nach einem der vorstehenden Ansprüche, wobei die Deckschicht (110) und insbesondere der erste und/oder der zweite Deckschichtbereich (120, 130) aus einem galvanisch abgeschiedenen Metall gefertigt sind.

6. Vorrichtung (100) nach einem der vorstehenden Ansprüche, wobei das erste antimikrobielle Edelmetall und/oder das zweite antimikrobielle Edelmetall aus einer Gruppe ausgewählt sind, welche Silber, Gold, Kupfer und Legierungen dieser Metalle umfasst.

7. Vorrichtung (100) nach einem der vorstehenden Ansprüche, wobei das erste antimikrobielle Edelmetall und das zweite antimikrobielle Edelmetall gleich sind.

8. Vorrichtung (100) nach einem der vorstehenden Ansprüche, wobei der Körper (105) in einer ersten Ebene einen Querschnitt aufweist, welcher eine erste Richtung (210) und eine hierzu orthogonale zweite Richtung (220) aufweist, wobei die Länge der ersten Richtung (210) länger als die Länge der zweiten Richtung (220) ist, und wobei vorzugsweise der Körper (105) in der ersten Ebene einen Querschnitt aufweist, der eine ovale Form und bevorzugt eine spitz-ovale Form aufweist.

9. Vorrichtung (100) nach Anspruch 8, wobei der Körper (105) in wenigstens einer weiteren Ebene, welche im Wesentlichen orthogonal zur ersten Ebene ist, einen trogförmigen Querschnitt aufweist.

10. Vorrichtung (100) nach Anspruch 8 oder 9, wobei für das Verhältnis *q* der Länge der zweiten Richtung (220) zur Länge der ersten Richtung (210) gilt 0,2 < *q* < 0,5 ist, vorzugsweise 0,3 < *q* < 0,4 ist, und bevorzugt *q* ≈ 0,35.

11. Vorrichtung (100) nach einem der vorstehenden Ansprüche, wobei sich der Körper (105) entlang einer ersten Richtung (210) longitudinal erstreckt und die Vertiefung (125) in einer hierzu im wesentlichen parallelen Richtung langgestreckt ist.

12. Vorrichtung (100) nach einem der vorstehenden Ansprüche, wobei der erste Deckschichtbereich (120) und der zweiten Deckschichtbereich (130) im Allgemeinen einander gegenüberliegend angeordnet sind.

13. Vorrichtung (100) nach einem der vorstehenden Ansprüche, wobei der Körper (105) eine Form aufweist, welche langgestreckt linsenförmig oder langestreckt ellipsoidal ist, und vorzugsweise allgemein spindelförmig ist.

14. Verwendung der Vorrichtung (100) nach einem der vorstehenden Ansprüche zur antimikrobiellen oder antibakteriellen Handreinigung.

## Claims

1. A hand cleaning device (100) comprising a body (105) having a cover layer (110), wherein the cover layer (110) comprises a first cover layer region (120) and a second cover layer region (130), and the first and second cover layer regions (120, 130) are shaped at least in part along a generally convex course, and wherein the first cover layer region defines a depression (125),
**characterized in that**
the first cover layer region (120) and the second cover layer region (130) are made of a first antimicrobial noble metal and a second antimicrobial noble metal, respectively, and
the body (105) is made of a material that does not contain any significant amounts of antimicrobial noble metals,
wherein the depression (125) defined by the first cover layer region (120) is defined by a deviation from the generally convex course of the first cover layer region (120), and preferably the first cover layer region (120) is shaped along a generally concave course in the region of the depression (125) and has only continuous radii of curvature.

2. The device of claim 1, wherein the cover layer (110) has a thickness in a range between 2µm and 15pm, preferably in a range between 3µm and 13pm, further preferably in a range between 4µm and 11µm, and more preferably in a range between 5µm and 9µm.

3. The device (100) of any of the preceding claims, wherein the body (105) is connected to the cover layer (110) by means of a support layer.

4. The device (100) of any one of the preceding claims, wherein the cover layer (110) substantially completely encloses the body (105).

5. The device (100) of any of the preceding claims, wherein the cover layer (110) and in particular the first and/or second cover layer regions (120, 130) are made of a galvanically deposited metal.

6. The device (100) of any one of the preceding claims, wherein the first antimicrobial noble metal and/or the second antimicrobial noble metal are selected from a group comprising silver, gold, copper, and alloys of these metals.

7. The device (100) of any one of the preceding claims, wherein the first antimicrobial noble metal and the second antimicrobial noble metal are the same.

8. The device (100) of any of the preceding claims, wherein the body (105), in a first plane, has a cross-section having a first direction (210) and a second direction (220) orthogonal thereto, wherein the length of the first direction (210) is longer than the length of the second direction (220), and wherein preferably the body (105) has a cross-section in the first plane having an oval shape and preferably an ogival-pointed shape.

9. The device (100) of claim 8, wherein the body (105) has a trough-shaped cross-section in at least one further plane that is substantially orthogonal to the first plane.

10. The device (100) of claim 8 or 9, wherein for the ratio *q* of the length of the second direction (220) to the length of the first direction (210), 0.2 < *q* < 0.5, preferably 0.3 < *q* < 0.4, and preferably *q* ≈ 0.35.

11. The device (100) of any of the preceding claims, wherein the body (105) extends longitudinally along a first direction (210) and the depression (125) is elongated in a direction substantially parallel thereto.

12. The device (100) of any one of the preceding claims, wherein the first cover layer region (120) and the second cover layer region (130) are arranged generally opposite each other.

13. The device (100) of any of the preceding claims, wherein the body (105) has a shape which is elongated in a lenticular shape or elongated in an ellipsoidal shape, and preferably is generally spindle-shaped.

14. Use of the device (100) of any of the preceding claims for antimicrobial or antibacterial hand cleaning.

## Revendications

1. Dispositif (100) destiné au nettoyage des mains, présentant un corps (105) avec une couche de recouvrement (110), sachant que la couche de recouvrement (110) présente une première zone de couche de recouvrement (120) et une seconde zone de couche de recouvrement (130), et que la première et la seconde zones de couche de recouvrement (120, 130) présentent au moins partiellement un tracé convexe général, et sachant que la première zone de couche de recouvrement définit un renfoncement (125),
**caractérisé en ce que**
la première zone de couche de recouvrement (120) et la seconde zone de couche de recouvrement (130) sont fabriquées en un premier métal précieux antimicrobien et/ou un second métal précieux antimicrobien, et **en ce que**
le corps (105) est fabriqué en une matière qui ne contient pas de quantités importantes de métaux précieux antimicrobiens,
sachant que le renfoncement (125) défini par la première zone de couche de recouvrement (120) est défini par une divergence du tracé convexe général de la première zone de couche de recouvrement (120) et que de préférence la première zone de couche de recouvrement (120) présente un tracé concave général dans la zone du renfoncement, et présente seulement des rayons de courbure continus.

2. Dispositif selon la revendication 1, sachant que la couche de recouvrement (110) présente une épaisseur qui est située dans une zone entre 2 µm et 15 µm, de préférence dans une zone entre 3 µm et 13 µm, davantage de préférence dans une zone entre 4 µm et 11 µm, et de préférence dans une zone entre 5 µm et 9 µm.

3. Dispositif (100) selon une quelconque des revendications précédentes, sachant que le corps (105) est relié à la couche de recouvrement (110) au moyen d'une couche support.

4. Dispositif (100) selon une quelconque des revendications précédentes, sachant que la couche de recouvrement (110) entoure intégralement le corps (105) pour l'essentiel.

5. Dispositif (100) selon une quelconque des revendications précédentes, sachant que la couche de recouvrement (110) et notamment la première et/ou la seconde zone de couche de recouvrement (120, 130) sont fabriquées en un métal déposé par voie galvanique.

6. Dispositif (100) selon une quelconque des revendications précédentes, sachant que le premier métal précieux antimicrobien et/ou le second métal précieux antimicrobien sont sélectionnés à partir d'un groupe qui comporte l'argent, l'or, le cuivre et des alliages de ces métaux.

7. Dispositif (100) selon une quelconque des revendications précédentes, sachant que le premier métal précieux antimicrobien et le second métal précieux antimicrobien sont identiques.

8. Dispositif (100) selon une quelconque des revendications précédentes, sachant que le corps (105) présente, à un premier niveau une section transversale, qui présente une première direction (210) et une seconde direction (220) orthogonale à celle-ci, sachant que la longueur de la première direction (210) est plus longue que la longueur de la seconde direction (220), et sachant que de préférence le corps (105) présente au premier niveau une section transversale qui présente une forme ovale et de préférence une forme ovale-pointue.

9. Dispositif (100) selon la revendication 8, sachant que le corps (105) présente, au moins sur un autre niveau qui est pour l'essentiel orthogonal au premier niveau, une section transversale en forme d'auge.

10. Dispositif (100) selon la revendication 8 ou 9, sachant que pour le rapport *q* de la longueur de la seconde direction (220) par rapport à la longueur de la première direction (210), l'équation 0,2 < *q* < 0,5 s'applique, de préférence 0,3 < *q* < 0,4 et de préférence *q* ≈ 0,35.

11. Dispositif (100) selon une quelconque des revendications précédentes, sachant que le corps (105) s'étend longitudinalement le long d'une première direction (210) et que le renfoncement (125) est étiré en longueur dans une direction parallèle à celle-ci pour l'essentiel.

12. Dispositif (100) selon une quelconque des revendications précédentes, sachant que la première zone de couche de recouvrement (120) et la seconde zone de couche de recouvrement (130) sont disposées en général face à face l'une de l'autre.

13. Dispositif (100) selon une quelconque des revendications précédentes, dans lequel le corps (105) présente une forme qui est étirée en longueur de manière lenticulaire allongée, ou est étirée en longueur de manière ellipsoïdale, et est de préférence généralement fusiforme.

14. Utilisation du dispositif (100) selon une quelconque des revendications précédentes en vue du nettoyage des mains antimicrobien ou antibactérien.
